# EUROPEAN PATENT SPECIFICATION

(11) **EP 1 175 878 B1**
(45) Date of publication and mention of the grant of the patent: **26.03.2003**
(21) Application number: 01123254.3
(22) Date of filing: 12.12.1995
(51) Int. Cl.: A61F 2/44, A61F 2/46

(54) **Conically-shaped fusion cage**
Konisch geformter Fusionskäfig
Cage de fusion de forme conique

(30) Priority: 12.12.1994 US 354364
(43) Date of publication of application: 30.01.2002
(62) Divisional of application: 95119558.5
(73) Proprietor: Howmedica Osteonics Corp., Allendale, New Jersey 07401-1677 (US)
(72) Inventor: Pavlov, Paul W., MD, 6522 JV Nijmegen (NL); Winslow, Charles J., Walnut Creek, CA 94595 (US); Jayne, Kirk, Alameda, CA 94501 (US); Klyce, Henry A., Piedmont, CA 94611 (US)
(74) Representative: HOFFMANN - EITLE

(56) References cited:
- EP-A- 0 307 241
- EP-A- 0 551 187
- EP-A- 0 637 440
- WO-A-87/07827
- WO-A-89/12431
- WO-A-91/06261
- WO-A-94/17759
- DE-A- 3 505 567
- DE-A- 4 302 397
- DE-C- 4 323 595
- FR-A- 2 350 824
- FR-A- 2 710 519

## Description

### BACKGROUND

### Field of the Invention

The present invention is directed to devices for facilitating the fusing of bone structures and more particularly the fusing together of adjacent vertebral bodies or bone structures.

### Background of the Invention

Technical literature and patent documents disclose a number of devices and methods for fusing bones together. One such device which has proven to be successful is disclosed in U.S. Patent 4,961,740, entitled "V-THREAD FUSION CAGE AND METHOD OF FUSING A BONE JOINT," which patent has been assigned the present assignee. The referenced patent discloses a fusion cage which is preferably cylindrical and has a thread formed as part of the external cylindrical surface. The fusion cage defines an internal cavity and apertures through the wall of the cage which communicate the external cylindrical surface with the internal cavity. The apertures are formed in the valleys of the thread. Normally two such cages are used to stabilized and fuse together adjacent vertebral bodies or bone structures.

In practice, using a posterior approach, a patient's vertebral bone structures are exposed and degenerate disk material located between the vertebral bone structures is removed. A threaded tap is used to tap a complementary thread in the upper and lower vertebral bone structures preparatory to the insertion of the above fusion cage. Once such tapping has been accomplished, using an introduction tool, the fusion cage is screwed into the space between the adjacent vertebral bone structures. The thread bites into the bone of the upper and lower vertebral bone structures, stabilizing the bone structures, and preventing the fusion cage from working out of this position due to patient movement. Generally two such fusion cages are applied using this technique. Once the two implants have been positioned, then bone growth inducing substances, such as bone chips, are packed into the internal cavity of the fusion cages. These bone growth inducing substances come into immediate contact with the bone from the vertebral bone structures which project into the internal cavity through the apertures. Such projection of bone is due to the fact that the apertures are formed in the valleys of the external thread of the fusion cage. Such immediate bone to bone contact between the vertebral bone structures and the bone pack within the fusion cages results in more rapid propagation of bone cells between the adjacent vertebral bone structures and thus a more rapid fusion of the adjacent vertebral bone structures.

It is to be understood that in the above method, bone growth inducing substances can be prepacked into the cages before the cages are implanted between the vertebral body structures.

EP 0 637 440, which is a prior art in terms of Art. 54(3)(4) EPC, discloses a hollow tubular intersomatic implant comprising a cage body, wherein the diameter of a first end is larger than the diameter of a second end to be implanted in the spine.

### Summary of the Invention

It is an object of the present invention to provide a fusion cage which has been designed to be implanted using principally a posterior approach to the vertebral bone structures.

This object is solved by a fusion cage as defined by claim 1.

Several embodiments are described in the sub-claims.

According to the present invention, the fusion cage includes a cage body having a proximal end and a distal end, said proximal end having a diameter which is smaller than the diameter of the distal end.

In an embodiment, the distal end further is rounded with for example a bull nose in order to facilitate the insertion of the cage body relative to one or more bone structures. The distal end could alternatively have a snub nose with or without a starter turn of a thread. The snub nose has a starter diameter that is smaller than the diameter of the distal end. The cage body is preferably conically-shaped. This shape is particularly advantageous due to the fact that the normal lordosis of the vertebral bone structures defines a wedged-shape space for a vertebral disk between, for example, lumbar vertebrae. Accordingly, the conically-shaped body cage can be sized and selected in order to maintain or enlarge upon the normal lordosis.

In a second embodiment of the present invention the cage body can include a cylindrically-shaped portion and a conically-shaped portion. The cylindrically-shaped portion is located adjacent to the distal end and the conically-shaped portion extends from the cylindrically-shaped portion and tapers toward the proximal end.

In a third embodiment of the present invention, the cage body has an outer surface and at least one flute formed in the outer surface. These flutes act as a relief much as the flute placed on self-tapping screws in order to facilitate the insertion of the fusion cage using a twisting motion between two vertebral bone structures.

According to the invention, the cage body has an outer surface and a thread formed as part of the outer surface. The thread aids the cage body in being inserted. As the cage is inserted, it gradually spreads apart the vertebral bone structures in order to regain or enlarge the natural lordosis of the adjacent vertebral bone structures. As with other embodiments of the present invention, flutes can be provided in the thread in order to allow for enhanced thread tapping by the cage and for a smoother insertion of the fusion cage between the vertebral bone structures. Preferably two or three flutes would be formed spaced about the fusion cage in order that one flute would be engaging with or adjacent to an upper vertebral bone structures with another flute being engaging with or adjacent to a lower vertebral bone structure. Such a relationship maintains alignment of the fusion cage and prevent wandering as the fusion cage is introduced between the two vertebral bone structures. Without two or more flutes, wandering might occur due to the fact that the thread is only substantially engaged with the vertebral bone structures and not with the disk material between the vertebral bone structures, which disk material does not provide support to the thread.

Any of the above embodiments can be provided with a plurality of apertures through the fusion cage and an internal cavity with the apertures communicating between the internal cavity and the external surface of the fusion cage. Bone growth inducing substances, such as bone chips, can be packed into the internal cavity either before the fusion cage is inserted or after the fusion cage has reached a final insertion position, or packed in both before and after. The bone chips come in contact with the vertebral bone structures through the apertures in order to facilitate fusion between the adjacent vertebral bone structures.

The cage body can have a round or bull nose distal end with one or more flutes formed in the round or bull nose distal end in order to enhance the self-tapping nature of the fusion cage and to prevent the cage from wandering.

Introduction tools allow the fusion cage to be accurately positioned between the vertebral bone structures. A preferred introduction tool allows for the cage to be implanted and thereafter allows an end cap of the cage to be conveniently removed, if desired, in order to place bone growth inducing substances in the cage.

A fusion cage as defined by the present invention is applicable in a method that affords access to adjacent vertebral bone structures using an posterior approach and procedure. Such posterior approach and procedure can be performed percutaneously using a minimally invasive technique with an introduction set including cannulas. Such a procedure is minimally invasive as the tissues can be spread using a set of cannula of increasing size and a small opening thereby developed through which a fusion cage can be inserted. Such a procedure is less traumatic to the tissue than an alternate posterior approach and procedure, also known as an posterior lumbar interbody fusion, where an incision is made, through the tissues. It is to be understood however that either posterior approach and procedure can be used with the fusion cage of the invention.

After such access, using preferably a minimally invasive technique, degenerate disk material can be removed and, using a cannula and insertion tool, an appropriately shaped fusion cage can be screwed into place between the vertebral bone structures in order to stabilize the vertebral bone structures and allow for fusion. Either preparatory to insertion of the fusion cage or after it has been inserted, bone chips or other bone growth inducing substances can be inserted into the fusion cage to promote bone to bone contact and subsequent fusion.

It is to be understood that although the above-embodiments have been described with respect to the fusion of adjacent vertebral bodies or bone structures, that the present invention can be used (1) to fuse together a variety of bone structures, in addition (2) to being fused to one bone structure and used as, for example, a base for an implant or (3) to being used to reunite the pieces of a broken bone.

Other objects and advantages of the invention can be obtained through a review of the specification and the figures.

### Brief Description of the Figure

Figure 1 is a partially sectional side view of an embodiment of the posterior fusion cage of the invention.
Figure 2 depicts a left end (distal end) view of the fusion cage of Figure 1.
Figure 3 depicts a right end (proximal end) view of the fusion cage of Figure 1.
Figure 4 depicts a view through line 20-20 of the fusion cage of Figure 1.
Figures 5, 6, and 7 depict progressive stages in the method of inserting the posterior fusion cage between adjacent vertebral bone structures using the cage depicted in Figure 9.
Figure 8 depicts a side view of an alternative embodiment of the posterior fusion cage of the invention.
Figure 9 depicts a side view of another embodiment of the posterior fusion cage of the invention.
Figure 10 depicts a left end (distal end) view of the embodiment of the fusion cage of Figure 9.
Figure 11 depicts the fusion cage of Figure 9 in conjunction with a new preferred insertion tool that can preferably be used with the posterior fusion cages of Figure 1 and 9.
Figure 12 depicts an end view of the insertion tool of Figure 11 along line 28-28.
Figure 13 depicts a partially broken away view of the fusion cage and the insertion tool of Figure 11 connected together.
Figure 14 depicts a perspective view of the end of the insertion tool depicted in Figure 12.
Figure 15 depicts a partially sectional view of the handle of the insertion tool of Figure 11.

### Detailed Description of the Preferred Embodiment

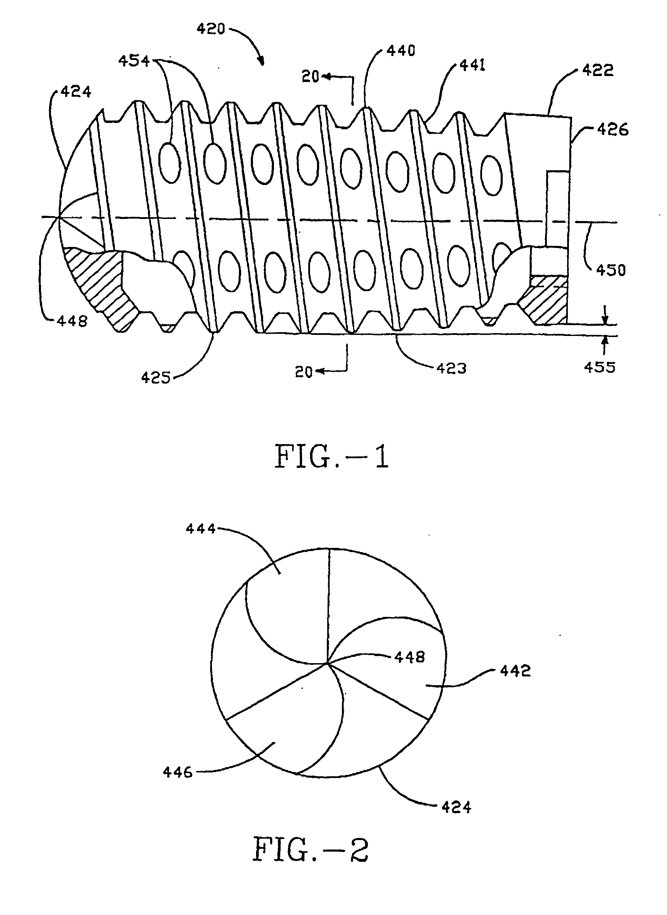

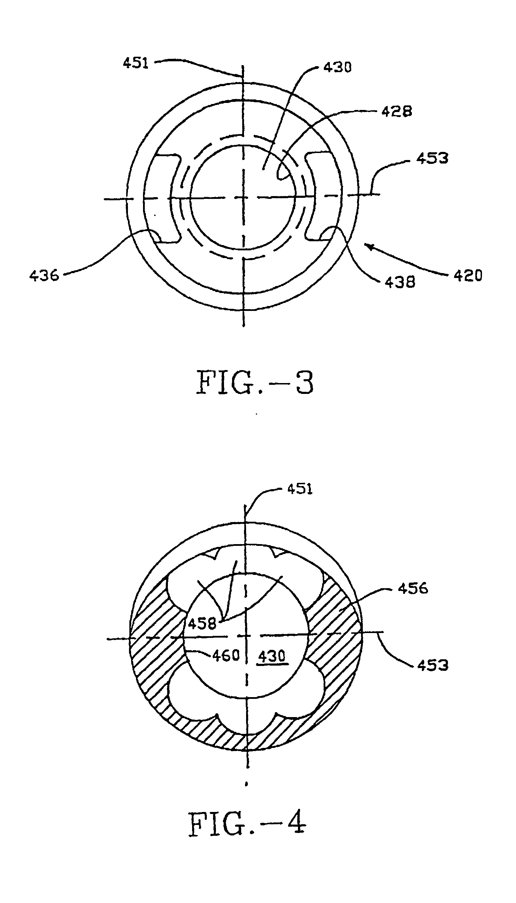

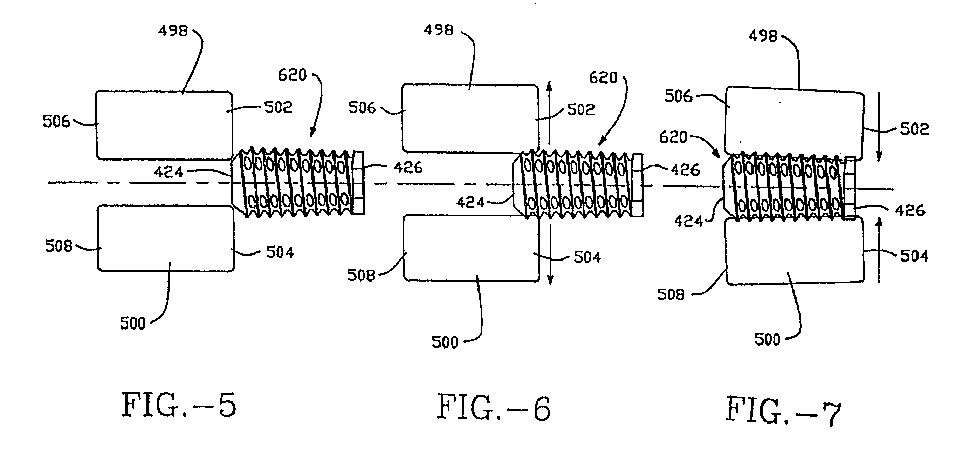

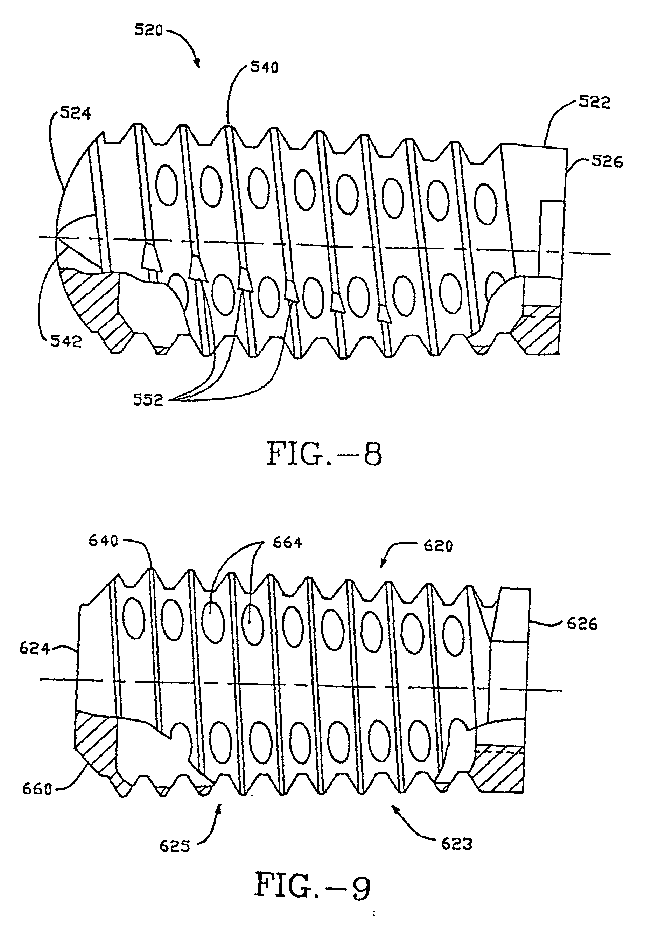

With respect to the figures in a particular Figure 1, a side view of the preferred embodiment of the posterior fusion cage 420 is depicted. Fusion cage 420 includes a fusion cage body 422 which in this preferred embodiment is provided with a conically-shaped portion 423 and a cylindrically-shaped portion 425. It is to be understood that alternatively the entire body 422 can be conically-shaped. Further, as appropriate the shape of the cage body 422 can be more complex with various conical and/or cylindrical configurations. Fusion cage 420 includes a distal end 424 and a proximal end 426. The distal end 424 in a preferred embodiment is rounded or bull nosed in order to facilitate the insertion of the fusion cage 420 relative to one or more bone structures. The proximal end 426 includes an opening 428 (Figure 3) which communicates with an internal cavity 430 (Figure 4) defined by the fusion cage 420. The opening 428 in a preferred embodiment is threaded so that it can receive an end cap or plug. End cap is used to close off the proximal end 426 and retain bone growth inducing substances, such as bone chips, packed therein as described herein-below. End cap includes a threaded bore which is designed to receive an insertion tool. The threaded bore has an initial unthreaded, square or hex-shaped portion which can be used with a socket wrench to tightly position end cap in opening 428 and which can also be engaged by the insertion tool of Figure 11 described below. The hex-shaped portion can be otherwise shaped as described above.

The proximal end 426 of the embodiment of Figure 3 further define first and second peripheral indentations 436, 438 which are centered about transverse axis 453. These peripheral indentations 436, 438 receive tangs from an insertion tool as described below for facilitating the insertion of the fusion cage 420 These identifications are also used to line up the cage 420 for proper insertion between the vertebral bodies as discussed below.

A thread 440 is defined as part of the outer cylindrical surface 441 of the body 422. It is to be understood that the thread can be replaced with a plurality of interrupted or discrete threads or a plurality of projections, ridges, protrusions, barbs, or spurs and be within the spirit and scope of the invention.

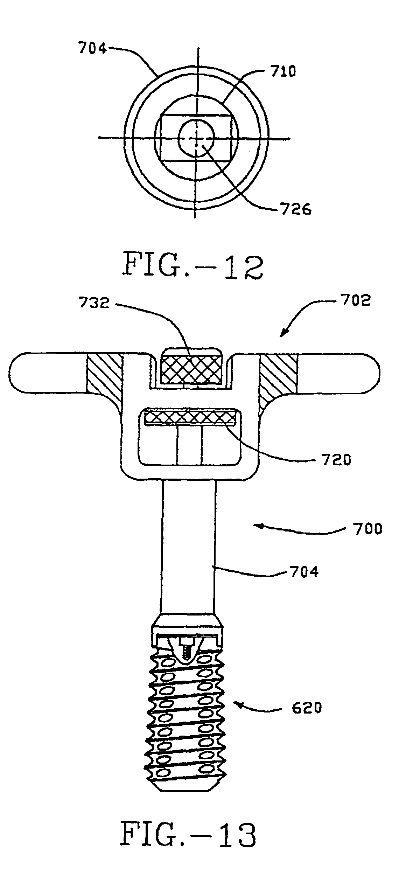

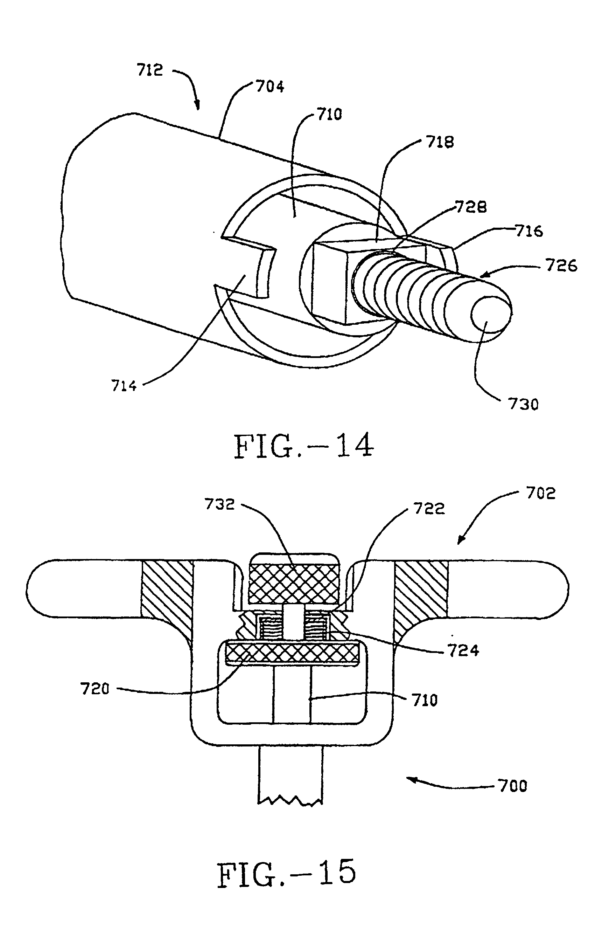

The rounded distal end 424, and at least some of the turns of thread 440 can in a preferred embodiment can define flutes or relief grooves 442, 444, and 446 (Figures 8, 9). It is to be understood that in alternative embodiments the flutes can be eliminated from the distal end 424 and the thread 440, since for example, the bore for the insertion of the fusion cage 420 between the vertebral bodies can be pre-tapped. Still in alternative embodiment, the flutes on the distal end can remain to assist in the insertion of the cage 420 between the vertebral bodies. In a preferred embodiment, flutes 442, 444, and 446 meet at a central point 448 of the distal end 424 on the longitudal axis 450 of the fusion cage 420. In other embodiments the flutes can be smaller and not extend all the way to the central point 448 on the longitude axis 450. Still as indicated above in other embodiments, the flutes can be eliminated from the distal end 424 and the thread 440 and such embodiments are still within the spirit and scope of the invention.

The flutes can extend from the distal end 424 toward the proximal end 426 as shown in the alternative embodiment in Figure 8 with respect to flute 542. These flutes are defined by the sections 552 which are removed from the thread. In this embodiment, the flutes become narrower as they approach the proximal end 526 due to the fact that thread relief for purposes of self-tapping becomes less important as the cage reaches a final resting position. As shown in other embodiments, the flutes can be deeper and extend from the distal end completely to the proximal end. Still further in other embodiments the flutes can be confined to the first several turns of the thread adjacent to the distal end and/or to just the distal end.

With respect to Figures 1, 4, a plurality of apertures 454 are provided through wall 456 of the fusion cage 420. In a preferred embodiment, these apertures 454 are formed by broaching grooves 458 in the internal surface 460 of the internal cavity 430. The effect of such broaching is to remove material from the valleys between the turns of the thread 440, thus defining the aperture 454. The advantages of such an arrangement are taught by the above-referenced U.S. Patent No. 4,961,740, and allows for immediate bone to bone contact between the vertebral bodies or bone structures and the bone packed within the internal cavity 430 of the fusion cage 420.

The apertures 454 in a preferred embodiment increase in size from smaller apertures closer to the proximal end 426 to a larger aperture closer to the distal end 424. This increase in size allows for more bone to bone contact. Alternatively in the embodiment as shown in Figure 1, all the apertures are of the same size.

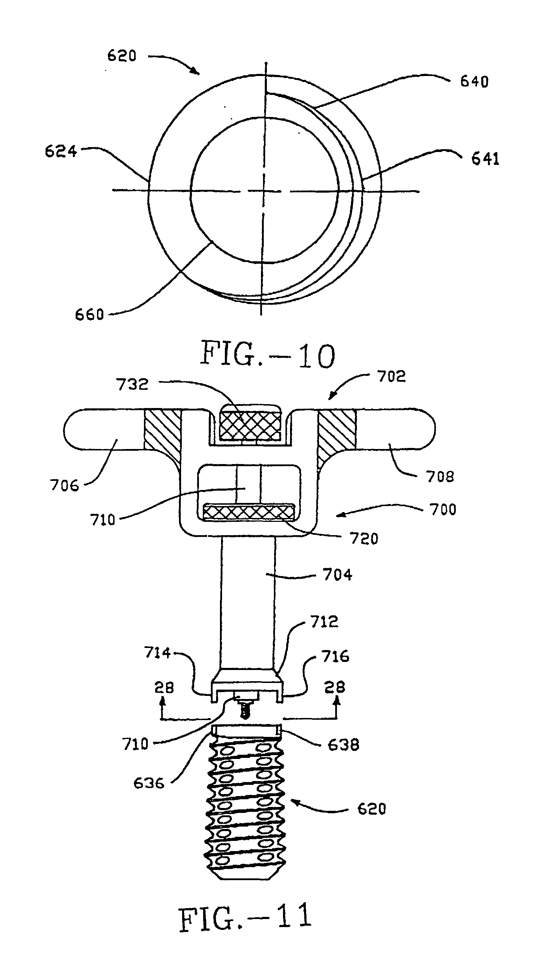

As can be seen in Figure 4, the apertures are clustered about a transverse axis 451, both at the upper and lower end of the axis. This is so that in position, the apertures come into contact with the upper and lower vertebral bone structures (Figure 7) to encourage bone growth through the fusion cage from the vertebral bone structures. The lateral sections of the fusion cage found along the other transverse axis 453 do not have apertures in order to prevent growth of disk material which might interfere with the bone fusing process. As can be seen viewing both Figures 3 and 4 together, the indentation 436 and 438 are centered on the axis 453 with the aperture 454 centered on axis 451. Axis 451 is preferably perpendicular to axis 453. The insertion tool has tangs that are inserted in indentation 436 and 438. Accordingly, the position of the insertion tool defines the position of the apertures 454 in that upon insertion the apertures 454 can be put in contact with the upper and lower vertebral bodies to allow bone ingrowth and prevent lateral ingrowth of disk material.

A preferred embodiment of the conically-shaped fusion cage 420 includes a fusion cage which is 28 millimeters in length having a distal end 424 with a diameter of 16 millimeters and a proximal end 426 with a diameter of 14 millimeters. The cage body is a right cylinder from the distal end 424 extending toward the proximal end 426 for four turns of thread 440. Then the cage 420 becomes a right cone from the remaining five turns of thread 440 until thread 440 terminates at proximal end 426. This conically-shaped portion is defined by relief 455 of 3.2°.. The thread has a pitch of 30° and there are ten turns per 2,54 cm (1 inch) with a thread depth of 0,153 cm (0.053 inches). Further the cage is made of a titanium metal or alloy such as Ti64. Preferably this and the other disclosed fusion cages disclosed are machined. However, the processes such as molding, casting or sintering can be used to accomplished formation of the fusion cages.

The cage is inserted between vertebral bodies using a preferred insertion tool 700 shown in Figure 11. Insertion tool 700 includes a handle 702 with an outer shaft 704 extending therefrom. The handle 702 includes first and second wings 706, 708 which make the handle easier to grab. The outer shank 704 is hollow and disposed within the outer shaft is an intermediate shaft 710 which can be seen extending from the cage engaging in 712 of the shaft 704. The cage engaging end 712 includes first and second tangs 714, 716 which can be inserted in the indentation of the cage such as for example indentations 436, 438, as shown in Figure 19, and indentations 636 and 638 shown in Figure 11. The end of shaft 710 includes a square-shaped drive 718 which engages the square-shaped unthreaded portion of the otherwise threaded bore such as the bore of the end cap. This same end cap can be used in the end of fusion cage 620. Alternatively, the square drive can be hexagonal shape with the unthreaded portion of the bore being hexagonal shaped to provide the necessary mating arrangement. Other mating shapes can also be used. A first knurled knob 720 is secured to immediate shaft 710 in order to provide a mechanism for rotating intermediate shaft 710 inside of outer shank 704. As can be seen in Figure 15, the intermediate shank 710 is spring biased relative to the handle 702 with a spring 722. Spring 722 is imbedded in a bore 724 of handle 702. In Figure 15, the first knurled knob 720 and the shank 710 are pulled back and thus compress the spring 722. In Figure 11, the first knurled knob 720 is released and the spring (not shown) is uncompressed.

An inner shaft 726 is located within a bore 728 of the intermediate shaft 710. The inner shaft 726 ends in a threaded portion 730 (Figure 14). The other end of inner shaft 726 is secured to the second knurled knob 732. Inner shaft 726 is free to rotate, through the use of the second knurled knob 72, within the bore 728 of the intermediate shaft 710. In addition the inner shaft 728 has limited freedom of motion along the longitudinal axis of the inner shaft 726.

The operation of the insertion tool 700 is as follows. With the insertion tool 700 not secured to a fusion cage, the insertion tool is as depicted in Figure 11 with the threaded portion 730 being either received entirely within bore 728 or extending a minimal amount out of bore 728. With the end cap secured in the fusion cage, the exposed square drive 718 is mated with the square portion of the bore in the end cap. The tangs 712, 714 are aligned with the indentations 636 and 638 and the tool is pushed in so that the tang 712, 714 are received in the indentations 636, 638. As this occurs, the knurled knob 730 moves up to the position as shown in Figure 13 and 15, compressing the spring. After this occurs, the second knurled knob 732 can be turned clockwise in order to engage the threaded portion 730 of the inner shaft 726 with the threaded portion of the bore of the end cap. This draws the fusion cage securely to the insertion tool 700 as shown in Figure 13. In this position, the cage is ready for insertion between the vertebral bodies. The handle 702 is then used to screw the cage between the vertebral bodies into the final resting position. Once the cage is in the final resting position, second knurled knob 732 is turned counter-clockwise in order to back the threaded 730 out of the threaded portion of the bore of the end cap. As this occurs, the spring 722 causes the square drive 718 to push against the end cap maintaining the end cap in its position relative to the fusion cage until the threaded portion 730 disengages itself from the threaded portion of the end cap, and the insertion tool 700 is disengaged from the fusion cage and can be removed. Thus the square drive, which is spring loaded, prevents the end cap on the cage from screwing out when the insertion tool is removed from the cage.

Should it be desired to move the end cap with the fusion cage in place, the square drive 718 can be inserted into the square portion of the threaded bore. The threaded portion 730 of the inner shaft 726 can then be screwed into engagement with the threaded portion of the threaded bore of the end cap, preferably with the tangs unaligned with the indentations. The first knurled knob 720 can then be turned in order to back the cap out of the fusion cage. A reverse of this operation can be used to insert the end cap into the fusion cage after additional bone growth inducing substances are packed into the fusion cage.

The method for inserting the fusion cage 420 of Figure 1 using a posterior approach and procedure to the vertebral bodies is as follows. Both a minimally invasive procedure and a more invasive procedure where a long incision is made in the back can be used.

With a posterior approach, using an introduction set such as described by way of example only, in U.S. Patent 4,863,430, entitled "INTRODUCTION SET WITH FLEXIBLE TROCAR WITH CURVED CANNULA,", but however with larger diameter instruments, an amount of disk material is removed between the two vertebral bodies or bone structures which are to be fused together. This procedure is accomplished through a cannula position adjacent to the vertebral bone structures. Then if required a thread is tapped in the upper and lower vertebral bodies. With the same or a larger diameter cannula, the fusion cage 420, or alternatively the preferred fusion cage 620 of Figure 9, can be introduced adjacent to the vertebral bone structures. In a first procedure, the fusion cage is packed with bone growth substances and the end cap is affixed to the fusion cage 620. Insertion tool 700 is then secured to the fusion cage 620 and the fusion cage is guided through the cannula to a location adjacent to the upper and lower vertebral body such as presented schematically in Figures 5, 6, 7, by upper body 498 and lower body 500. In the initial position as shown in Figure 5, the fusion cage 620 is adjacent to the posterior sections 502, 504 of the vertebral bodies 498, 500. As the introduction tool is turned, the thread 640 (Figure 9) of the fusion cage 620 bites into the vertebral bodies 498, 500. Further turning of the introduction tool causes the fusion cage to move through the position shown in Figure 6 to the final resting position shown in Figure 7, where the distal end 624 is moved adjacent to the anterior sections 506, 508 of the vertebral bone structures 498, 500. As this occurs, the fusion cage 620 increases the lordosis or spacing between the vertebral bodies, basically distracting the vertebral bodies. It is noted that most of the distraction occurs adjacent to the anterior sections, but that distraction also occur at the posterior sections. Preferably, the lordosis is increased over the normal lordosis in order to stabilize the vertebral bone structures prior to fusion occurring. Stabilization occurs due to the fact that increased lordosis places additional stress on the anterior longitudinal ligaments which are part of the anatomy holding the vertebral bodies in place.

Once the fusion cage 620 is appropriately positioned, the insertion tool 700 is unscrewed from the cap and the insertion tool 700 is pulled away from the fusion cage.

It is to be understood that the cage can be implanted without the use of a cannula by making a larger incision in the back. With this arrangement the bone chips would more often be packed into the cage after the cage reaches its final position and then an end cap would be secured t the cage. In the final position apertures 454 or 664 (embodiment of Figure 9) would be positioned adjacent vertebral bodies 498 and 500. No matter which procedure is used to insert the cage 420 or 620, it is advantageous to provide a bore between the vertebral bodies that is less than the diameter of the distal end 424. Thus, for a cage 420 or a cage 620 with a distal end having an 18 diameter, the bore would be 14 millimeters. Inserting the cage 420 or the cage 620 would cause the vertebral bodies to be distracted (Figure 6) and then rock back (Figure 7) onto the conically-shaped portion of the fusion cage 420.

An alternative embodiment of a fusion cage 520 is shown in Figure 8. Fusion cage 520 includes a distal end 524 and an a proximal end 526. A thread 540 is defined on the external surface of the fusion cage 520. Defined by the thread 540 are flutes 542, 544, 546, which in this embodiment extend from the distal end 524 toward the proximal end 526. These flutes provide thread relief 552 allowing the fusion cage 520 to be self-tapping.

Still an alternative and preferred embodiment of the invention as mentioned above is shown in Figure 9. In this embodiment the fusion cage 620 includes a blunt or flat distal end 624 and a proximal end 626. As in the other embodiments, the fusion cage is conically-shaped, and includes a thread 640 and aperture 654.

Figure 10 includes a view of the distal end 624 of the fusion cage 620. This distal end 624 uses a snub nosed portion that is closed. The diameter of the snub nosed portion 660 is smaller than the largest root of the thread 640 at the distal end 624. As can be seen in Figure 10, the thread 640 has a starter portion or starter turn 641 which includes approximately the first half turn of the thread 640. The diameter of the starter portion 641, as can be seen Figure 10, is substantially less than the outside diameter of the four turns of thread 640 which comprised the cylindrical portion 625. From the cylindrical portion 625, the cage 620 and the thread 640 taper off to the proximal end 626 and define the conically-shaped portion 623.

The starter turn 641 of thread 640, as the name implies, assist in promoting the proper engagement of the thread 640 with the upper and lower vertebral bodies. In this embodiment, as in prior embodiments, the distal end has a diameter of approximately 16 millimeters. The diameter of the snub nosed portion 660 is about 10 millimeters.

### Industrial Applicability

The present invention affords the advantages of a fusion cage which can be introduced through a posterior approach in order to maintain or increase lordosis between adjacent vertebral bodies. The fusion cage has the advantage of being conically- shaped and self-tapping through the use of external flutes. The flutes additionally assist in keeping the fusion cage aligned and centered as the cage is being inserted between the vertebral bone structures.

Other, advantages, aspects, and objects of the invention can be obtained through a review of the claims and the appended .figures.

Additional embodiments of the invention can be constructed and fall within the scope of the claims.

## Claims

1. A fusion cage (420) for promoting fusion with one or more bone structures comprising:
a cage body (422) having a proximal trailing end (426) and a distal leading end (424), said proximal end having a diameter which is smaller than a diameter of said distal end,
said cage body having an outer surface (441) and a thread (440) formed into said outer surface.

2. A fusion cage as claimed in claim 1, said cage body has at least one flute (442) formed in the outer surface.

3. The fusion cage of claims 1 or 2, wherein:
said cage has a cylindrically-shaped portion (425) located adjacent to the distal end and a conically-shaped portion (423) located adjacent to the proximal end.

4. The fusion cage of claim 3 wherein:
said thread is defined by the cylindrically-shaped portion and the conically-shaped portion.

5. The fusion cage of any one of the preceding claims wherein:
said cage body has at least one flute (442) formed in the thread.

6. The fusion cage of claim 5 wherein the flute is formed in the distal end in order to facilitate the insertion of the fusion cage in the one or more bone structures, the flute extending from the distal end toward the proximal end.

7. The fusion cage of any one of the preceding claims including:
at least three flutes (442,444,446) formed in the outer surface being equally spaced about said distal end.

8. The fusion cage of any one of the preceding claims wherein:
said cage body has an internal cavity (430); and
a plurality of apertures (454) are formed through the cage body which communicate said outer surface with said internal cavity.

9. The fusion cage of any one of the preceding claims wherein said cage body is a right circular cone.

10. The fusion cage of any one of the preceding claims further comprising a thread with a plurality of turns found on the outer surface, and the flute is formed in at least one of said turns.

11. The fusion cage of any one of the preceding claims in combination with an insertion tool (700), said fusion cage including:
said proximal end having an opening (428) which communicates with an internal cavity (430);
an end cap which can fit into said opening in order to close off said internal cavity;
said proximal end including at least one insertion tool-receiving indentation (636,638);
said end cap including an insertion tool receiving threaded bore with an unthreaded portion with an irregularity; and
said insertion tool including:
a tang (714,716)for being received in said indentation and a threaded shaft for being received in said threaded bore, and a shaft for mating with the unthreaded portion, said insertion tool for being engaged with said fusion cage for inserting said fusion cage relative to the one or more bone structures.

12. The fusion cage of claim 8 including:
said apertures increase in size from the distal end toward the proximal end.

13. A fusion cage as claimed in any one of the preceding claims:
said cage body having a longitudinal axis (450), and a first transverse axis (451) which is perpendicular to the longitudinal axis and a second transverse axis (453) which is perpendicular to both the longitudinal axis and the first transverse axis;
a position indicator located at said proximal end, which position indicator is located along the first transverse axis; and
said cage body having a plurality of apertures (454) for promoting bone growth into the cage body, which apertures are located only substantially along the second transverse axis between the proximal end and the distal end.

14. The fusion cage of claim 13 wherein:
said position indicator includes an indentation (636, 638) into said proximal end.

15. The fusion cage of claim 14 wherein:
said distal end has a snub nose (660) extending therefrom in order to facilitate insertion relative to one or more bone structures the snub nose having a diameter which is less than the diameter of the distal end (624).

16. The fusion cage of claim 15 wherein:
the cage body thread has a starter turn (641) located at the distal end, the starter turn of the thread extends from the snub nose in order to facilitate insertion relative to the one or more bone structures.

17. A fusion cage as claimed in any one of claims 1 to 10 and 12, in combination with an insertion tool (700) comprising:
said fusion cage proximal end including at least one insertion tool-receiving indentation (636,638);
said proximal end including an insertion tool-receiving threaded bore having an unthreaded portion with at least one irregularity; and
said insertion tool having a first shaft (704) with a tang (714,716) that can be received in said indentation, a second shaft (710) with a portion (718) which can mate with the unthreaded portion of the bore with the irregularity, and a third shaft (726) with a threaded bore (730) which can mate with the threaded bore.

18. The fusion cage and insertion tool combination of claim 17 wherein:
said second and said third shafts can rotate relative to the first shaft and relative to each other.

19. The fusion cage and insertion tool combination of claim 18 wherein:
one of said second and third shafts is biased relative to the other of said second and third shafts.

20. The fusion cage and insertion tool combination of claim 19 wherein:
said fusion cage includes an end cap which in part comprises said proximal end, and wherein said end cap includes said threaded bore, and wherein said end cap can be selectively removed from the remainder of the proximal end.

## Patentansprüche

1. Fusionskäfig (420) zum Fördern der Fusion mit einer oder mehreren Knochenstrukturen, umfassend:
einen Käfigkörper (422) mit einem proximalen hinteren Ende (426) und einem distalen vorderen Ende (424), wobei das proximale Ende einen Durchmesser aufweist, der kleiner als ein Durchmesser des distalen Endes ist, wobei der Käfigkörper eine Außenfläche (441) aufweist und ein Gewinde (440) in der Außenfläche ausgebildet ist.

2. Fusionskäfig nach Anspruch 1, wobei der Käfigkörper mindestens eine Nut (442) aufweist, die in der Außenfläche ausgebildet ist.

3. Fusionskäfig nach Anspruch 1 oder 2, wobei der Käfig einen zylinderförmigen Abschnitt (425), welcher dem distalen Ende benachbart angeordnet ist und einen kegelförmigen Abschnitt (423), welcher dem proximalen Ende benachbart angeordnet ist, aufweist.

4. Fusionskäfig nach Anspruch 3, wobei das Gewinde durch den zylinderförmigen Abschnitt und den kegelförmigen Abschnitt bestimmt ist.

5. Fusionskäfig nach einem der vorstehenden Ansprüche, wobei der Käfigkörper mindestens eine Nut (442) aufweist, die in dem Gewinde ausgebildet ist.

6. Fusionskäfig nach Anspruch 5, wobei die Nut in dem distalen Ende ausgebildet ist, um die Einführung des Fusionskäfigs in die eine oder die mehreren Knochenstrukturen zu erleichtern, die Nut sich von dem distalen Ende zu dem proximalen Ende hin erstreckt.

7. Fusionskäfig nach einem der vorstehenden Ansprüche, umfassend mindestens drei Nuten (442, 444, 446), die in der Außenfläche ausgebildet und um das distale Ende herum gleichmäßig zueinander beabstandet sind.

8. Fusionskäfig nach einem der vorstehenden Ansprüche, wobei der Käfigkörper einen inneren Hohlraum (430) aufweist und mehrere Öffnungen (454) durch den Käfigkörper ausgebildet sind, die die Außenfläche mit dem inneren Hohlraum verbinden.

9. Fusionskäfig nach einem der vorstehenden Ansprüche, wobei der Käfigkörper ein gerader Kreiskegel ist.

10. Fusionskäfig nach einem der vorstehenden Ansprüche, ferner umfassend ein Gewinde mit mehreren Windungen auf der Außenfläche, und wobei die Nut in mindestens einer der Windungen ausgebildet ist.

11. Fusionskäfig nach einem der vorstehenden Ansprüche, in Kombination mit einem Einführwerkzeug (700), wobei der Fusionskäfig umfasst:
das proximale Ende mit einer Öffnung (428), die mit einem inneren Hohlraum (430) in Verbindung steht;
eine Endabdeckung, die in die Öffnung eingepasst werden kann, um den inneren Hohlraum zu verschließen;
das proximale Ende mindestens eine Aussparung (636, 638) zum Aufnehmen des Einführwerkzeuges umfasst;
die Endabdeckung eine Gewindebohrung zum Aufnehmen des Einführwerkzeuges mit einem gewindelosen Abschnitt mit einer Unregelmäßigkeit umfasst; und wobei das Einführwerkzeug umfasst:
einen Vorsprung (714, 716), der von der Aussparung aufgenommen wird und einen Gewindeschaft, der in der Gewindebohrung aufgenommen wird, und einen Schaft zum Verbinden mit dem gewindelosen Abschnitt, wobei das Einführwerkzeug mit dem Fusionskäfig zum Einführen des Fusionskäfigs relativ zu der einen oder den mehreren Knochenstrukturen in Eingriff bringbar ist.

12. Fusionskäfig nach Anspruch 8, umfassend, dass die Öffnungen in ihrer Größe von dem distalen Ende zu dem proximalen Ende hin zunehmen.

13. Fusionskäfig nach einem der vorstehenden Ansprüche, wobei der Käfigkörper eine Längsachse (450) und eine erste Querachse (451) aufweist, die senkrecht zu der Längsachse und einer zweiten Querachse (453) ist, welche senkrecht zu beiden, der Längsachse und der ersten Querachse, ist;
ein Positionsindikator an dem proximalen Ende angeordnet ist, welcher Positionsindikatior entlang der ersten Querachse angeordnet ist; und
der Käfigkörper mehrere Öffnungen (454) zum Fördern des Knochenwachstums in den Käfigkörper aufweist, welche Öffnungen im wesentlichen nur entlang der zweiten Querachse zwischen dem proximalen Ende und dem distalen Ende angeordnet sind.

14. Fusionskäfig nach Anspruch 13, wobei der Positionsindikator eine Aussparung (636, 638) in dem proximalen Ende umfasst.

15. Fusionskäfig nach Anspruch 15, wobei das distale Ende eine Stupsnase (660) aufweist, die sich von diesem erstreckt, um die Einführung relativ zu der einen oder den mehreren Knochenstrukturen zu erleichtern, und wobei die Stupsnase einen Durchmesser aufweist, der geringer als der Durchmesser des distalen Endes (624) ist.

16. Fusionskäfig nach Anspruch 15, wobei das Käfigkörpergewinde eine Anfangswindung (641) aufweist, die an dem distalen Ende angeordnet ist, sich die Anfangswindung des Gewindes von der Stupsnase erstreckt, um die Einführung relativ zu der einen oder den mehreren Knochenstrukturen zu erleichtern.

17. Fusionskäfig nach einem der Ansprüche 1 bis 10 und 12, in Kombination mit einem Einführwerkzeug (700), umfassend:
mindestens eine Aussparung (636, 638) zum Aufnehmen eines Einführwerkzeuges in dem proximalen Ende des Käfigkörpers;
eine Gewindebohrung in dem proximalen Ende zum Aufnehmen eines Einführwerkzeuges mit einem gewindelosen Abschnitt mit mindestens einer Ungleichmäßigkeit; und
dem Einführwerkzeug mit einem ersten Schaft (704) mit einem Vorsprung (714, 716), der in der Aussparung aufnehmbar ist, einem zweiten Schaft (717) mit einem Abschnitt (718), der mit dem gewindelosen Abschnitt der Bohrung mit den Ungleichmäßigkeiten zusammenbringbar ist, und einem dritten Schaft (726) mit einer Gewindebohrung (730), die mit der Gewindebohrung zusammenbringbar ist.

18. Fusionskäfig und Einführwerkzeug-Kombination nach Anspruch 17, wobei sich der zweite und der dritte Schaft relativ zu dem ersten Schaft und relativ zueinander drehen können.

19. Kombination aus Fusionskäfig und Einführwerkzeug nach Anspruch 18, wobei einer der beiden zweiten und dritten Schäfte relativ zu dem anderen der beiden zweiten und dritten Schäfte vorgespannt ist.

20. Kombination aus Fusionskäfig und Einführwerkzeug nach Anspruch 19, wobei der Fusionskäfig eine Endabdeckung umfasst, die teilweise das proximale Ende umfasst und wobei die Endabdeckung die Gewindebohrung aufweist und wobei die Endabdeckung wahlweise von dem verbliebenen Bereich des proximalen Endes entfernt werden kann.

## Revendications

1. Cage de fusion (420) pour encourager la fusion avec une ou plusieurs structures osseuses comprenant :
un corps de cage (422) présentant une extrémité proximale arrière (426) et une extrémité distale avant (424), ladite extrémité proximale ayant un diamètre qui est plus petit que le diamètre de ladite extrémité distale, ledit corps de cage présentant une surface externe (441) et un filet (440) formé dans ladite surface externe.

2. Cage de fusion selon la revendication 1, où ledit corps de cage présente au moins une cannelure (442) formée dans la surface externe.

3. Cage de fusion selon les revendications 1 ou 2, où :
ladite cage présente une portion de forme cylindrique (425) située pour être adjacente à l'extrémité distale et une portion de forme conique (423) située pour être adjacente à l'extrémité proximale.

4. Cage de fusion selon la revendication 3, où :
ledit filet est défini par la portion de forme cylindrique et la portion de forme conique.

5. Cage de fusion selon l'une des revendications précédentes où :
ledit corps de cage présente au moins une cannelure (442) formée dans le filet.

6. Cage de fusion selon la revendication 5, où la cannelure est formée dans l'extrémité distale pour faciliter l'insertion de la cage de fusion dans une ou plusieurs structures osseuses, la cannelure s'étendant de l'extrémité distale vers l'extrémité proximale.

7. Cage de fusion selon l'une des revendications précédentes incluant :
au moins trois cannelures (442, 444, 446) formées dans la surface externe qui sont espacées uniformément autour de ladite extrémité distale.

8. Cage de fusion selon l'une des revendications précédentes où :
ledit corps de cage présente une cavité interne (430) ; et
plusieurs ouvertures (454) sont ménagées à travers le corps de cage qui mettent en communication ladite surface externe avec ladite cavité interne.

9. Cage de fusion selon l'une des revendications précédentes, où ledit corps de cage est un cône circulaire droit.

10. Cage de fusion selon l'une des revendications précédentes, comprenant en outre un filet avec plusieurs tours sur la surface extérieure, et la cannelure est formée dans au moins l'un desdits tours.

11. Cage de fusion selon l'une des revendications précédentes en combinaison avec un outil d'insertion (700), ladite cage de fusion comprenant :
ladite extrémité proximale présentant une ouverture (428) qui communique avec une cavité interne (430) ;
un capuchon d'extrémité qui peut s'adapter à ladite ouverture pour fermer ladite cavité interne ;
ladite extrémité proximale incluant au moins une encoche de réception d'outil d'insertion (636, 638) ;
ledit capuchon d'extrémité incluant un perçage fileté de réception d'outil d'insertion avec une portion non filetée avec une irrégularité ; et
ledit outil d'insertion incluant :
un fût (714, 716) destiné à être reçu dans ladite encoche et un arbre fileté destiné à être reçu dans ledit perçage fileté, et une tige pour correspondre à la portion non filetée, ledit outil d'insertion étant destiné à s'engager dans ladite cage de fusion pour insérer ladite cage de fusion relativement à une ou plusieurs structures osseuses.

12. Cage de fusion selon la revendication 8, incluant :
lesdites ouvertures augmentent en taille de l'extrémité distale vers l'extrémité proximale.

13. Cage de fusion selon l'une des revendications précédentes ;
ledit corps de cage ayant un axe longitudinal (450), et un premier axe transversal (451) qui est perpendiculaire à l'axe longitudinal et un second axe transversal (453) qui est perpendiculaire à la fois à l'axe longitudinal et au premier axe transversal ;
un indicateur de position situé à ladite extrémité proximale, cet indicateur de position étant localisé le long du premier axe transversal ; et
ledit corps de cage présentant plusieurs ouvertures (454) pour encourager la croissance de l'os dans le corps de cage, ces ouvertures se trouvant seulement sensiblement le long du second axe transversal entre l'extrémité proximale et l'extrémité distale.

14. Cage de fusion selon la revendication 13 où :
ledit indicateur de position comporte une encoche (636, 638) dans ladite extrémité proximale.

15. Cage de fusion selon la revendication 14 où :
ladite extrémité distale présente un nez camus (660) s'étendant à partir de celle-ci pour faciliter l'insertion relativement à une ou plusieurs structures osseuses, le nez camus ayant un diamètre qui est plus petit que le diamètre de l'extrémité distale (624).

16. Cage de fusion selon la revendication 15 où :
le filet du corps de cage présente un tour de départ (641) situé à l'extrémité distale, le tour de départ du filet s'étend depuis le nez camus pour faciliter l'insertion relativement à une ou plusieurs structures osseuses.

17. Cage de fusion selon l'une des revendications 1 à 10 et 12, en combinaison avec un outil d'insertion (700) comprenant :
ladite extrémité proximale de la cage de fusion incluant au moins une encoche de réception d'outil d'insertion (636, 638) ;
ladite extrémité proximale incluant un perçage fileté de réception d'outil d'insertion ayant une portion non filetée avec au moins une irrégularité ; et
ledit outil d'insertion présentant une première tige (704) avec un fût (714, 716) qui peut être reçu dans ladite encoche, une deuxième tige (710) avec une portion (718) qui peut correspondre à la portion non filetée du perçage avec l'irrégularité, et une troisième tige (726) avec un perçage fileté (730) qui peut correspondre au perçage fileté.

18. Combinaison de cage de fusion et d'outil d'insertion selon la revendication 17 où :
lesdits deuxième et troisième tiges peuvent tourner relativement à la première tige et l'une relativement à l'autre.

19. Combinaison de cage de fusion et d'outil d'insertion selon la revendication 18 où :
l'une desdites deuxième et troisième tiges est sollicitée relativement à l'autre desdites deuxième et troisième tiges.

20. Combinaison de cage de fusion et d'outil d'insertion selon la revendication 19 où :
ladite cage de fusion comporte un capuchon d'extrémité qui comprend en partie ladite extrémité proximale, et où ledit capuchon d'extrémité comporte ledit perçage fileté, et où ledit capuchon d'extrémité peut être retiré sélectivement du restant de l'extrémité proximale.
